(19) Europäisches Patentamt
European Patent Office
Office européen des brevets

(11) Publication number: **0 314 240 A2**

(12) **EUROPEAN PATENT APPLICATION**

(21) Application number: 88202336.9

(22) Date of filing: 19.10.88

(51) Int. Cl.[4]: C12P 21/00 , C07K 3/12 , //A61K39/29

(30) Priority: 26.10.87 US 113583

(43) Date of publication of application: 03.05.89 Bulletin 89/18

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Yamazaki, Shigeko
1279 Schwab Road
Hatfield PA, 19440(US)

(74) Representative: Hesketh, Alan, Dr.
European Patent Department Merck & Co., Inc. Terlings Park Eastwick Road
Harlow Essex, CM20 2QR(GB)

(54) **Process for purifying recombinant hepatitis antigens.**

(57) Methods of solubilizing recombinant surface antigen of hepatitis B virus are disclosed. In one protocol, solubilization is achieved with buffer, urea and dithiothreitol.

EP 0 314 240 A2

# PROCESS FOR PURIFYING RECOMBINANT HEPATITIS ANTIGENS

## INTRODUCTION

Hepatitis B surface antigen often occurs as a glycoprotein-protein complex that confers immunity against the pathological effects of subsequent infection by hepatitis B virus. Sources for safe, rapid and inexpensive manufacture of the antigen for vaccination purposes have been limited to serum or plasma from patients who synthesize large quantities of the requisite antigen, usually in the form of 22 nm particles. With the advent of recombinant DNA tech niques, DNA coding for the surface antigen is inserted into yeast, E. coli and other cellular systems, then expressed. The resulting polypeptide product varies substantially with the DNA construction used as well as the host or cellular systems employed to express the inserted DNA.

The HB virion is composed of two groups of structural proteins, the core proteins and the envelope or surface ("S") proteins. In addition to being the major surface proteins of the virion, i.e., Dane particle, the "S" proteins are the sole constituents of Australia antigen, or 22 nm particles. The "S" proteins are the translational products of a large open reading frame (ORF) encoding 389-400 amino acids, depending upon serotype. This ORF is demarcated into three domains, each of which begins with an ATG codon that is capable of functioning as a translational initiation site in vivo. These domains are referred to as pre-S1 (108-119 amino acids), pre-S2 (55 amino acids), and S (226 amino acids) in their respective 5′-3′ order in the gene. The six protein products derived from this ORF have the following compositions:

1) gp42 (42,000 dalton glycoprotein) = pre-S1/S2/S (meaning pre-S1 contiguous with pre-S2, contiguous with S),
2) p39 (p = protein) = pre-S1/S2/S,
3) gp36 = pre-S2/S (two glycosylation sites),
4) gp33 = pre-S2/S (one glycosylation site),
5) gp27 = S (one glycosylation site),
6) p24 = S.

All six proteins are present to an approximately equimolar extent in the HBV Dane particle. In the 22 nm particle, the 4 smaller proteins are present to an approximately equimolar extent, while gp42 and p39 are present in at most one or a few molecules per particle. The pre-S1 and pre-S2 regions may have functions of promoting secretion of the S region. For reviews of these fundamental properties of the protein, see Tiollais, P. et al., Science 213, 406 (1981) and Milich, D. R. et al., Proc. Natl. Acad. Sci. 82, 8168 (1985).

The pre-S2 region of the hepatitis B antigen comprises about 55AA (amino acid) residues. Its presence provides a dominant epitope that is more immunogenic in vivo than epitopes of the S protein, according to Neurath, A. R. et al., Science 224, 392 (1984), Neurath, A. R. et al., Nature 315, 154 (1985), and Milich, D. R. et al., supra. The pre-S2 polypeptide also has receptor-like properties for polymerized human serum albumin (pHSA), a trait also possessed by liver cells which are known to bind pHSA, Machida, A. et al., Gastroenterology 86, 910 (1984).

Since the presence of pre-S2 sequences in the surface antigen is a desirable property for the purposes of immunization, expression systems have been developed for the expression of pre-S1/S2/S protein and other variants. See, for example, U.S. application no. 824,405, filed January 31, 1986.

The methods of the present invention relate to solubilizing precipitates of recombinant surface antigen of hepatitis B virus (rHbsAg). Partially purified rHbsAg precipitates are solubilized by a combined and simultaneous treatment of urea and DTT. Precipitates or other insoluble entities frequently occur in the process of purifying rHbsAg from yeast and other expression systems. The present invention provides methods of rendering soluble such precipitates for the purposes of making a better and cheaper vaccine against hepatitis-associated diseases.

The technical advantages of solubilizing the partially purified recombinant surface antigen with denaturing agents in the presence of reducing agents include higher yield and greater purity. The solubilized product is also more stable. The technique of solubilization is rapid and simple.

Purification of recombinant proteins not infrequently requires new methods or novel combinations of old methods, since the composition of the source for recombinant forms e.g. yeast are different from conventional sources e.g. serum. One cannot predict what methods are useful for isolating proteins from recombinant cell cultures on the basis of the knowledge and know-how of methods useful for isolating proteins from classical or otherwise conventional sources. Purification processes designed for preparing vaccines require unusual purity in the product, another indication of the unpredictability in the art. For a

similar view, see U.S. Patent 4,624,918.

## BRIEF DESCRIPTION OF THE INVENTION

In this invention, there is disclosed a method for solubilizing precipitates of recombinant hepatitis B surface antigen from a precipitated partially purified recombinant hepatitis antigen obtained from a recombinant expression system, comprising the steps of:

(a) treating a preparation of precipitated, partially purified recombinant hepatitis antigen, or portion of said antigen thereof, with a quantity of denaturing agent in the presence of a reducing agent effective to solubilize said preparation, and

(b) removing the denaturing agent and the reducing agent, yielding a preparation of a substantially soluble, partially purified recombinant hepatitis antigen, or portion of said antigen thereof.

## DEFINITIONS AND ABBREVIATIONS

Specific activity: a weight-to-weight ratio of immunochemically reactive sAg to total protein.

| | |
|---|---|
| AA | amino acid |
| DTT | dithiothreitol |
| $ED_{50}$ | 50% effective dose |
| EDTA | ethylenediamine tetracetic acid trisodium salt |
| L | liter |
| Mr | mobility |
| MW | molecular weight |
| NMW | nominal molecular weight cutoff |
| pHSA | polymerized human serum albumin |
| PBS | phosphate buffered saline, 0.15M NaCl in 7mM sodium phosphate buffer, pH about 7.2 |
| PMSF | phenylmethylsulfonyl fluoride |
| psi | pounds per square inch |
| rHbsAg | recombinant hepatitis B surface antigen |
| S | surface |
| SDS-PAGE | sodium dodecyl sulfate polyacrylamide gel electrophoresis |

## DETAILED DESCRIPTION OF THE INVENTION

The processes of the present invention involve methods of solubilizing recombinant hepatitis B surface antigen (rHbsAg) from yeast extracts. These methods involve the combined treatment of rHbsAg with denaturing agent in the presence of reducing agent.

It will be understood that the novel purification processes of the present invention are applicable to a range of expressed rHbsAg including recombinant S protein or recombinant pre-S1/S2/S protein. One principle example is rHbsAg produced by yeast cells transformed by yeast vectors of Example 1. This system expresses an S amino acid sequence. Processes for the purification of other variant amino acid sequences of the S protein are encompassed by the present invention. The processes of the present invention are designed to provide rapid and efficient methods of purifying any rHbsAg, including S protein variants as well as pre-S1/S2/S protein variants, in accordance with the principles of the present invention. For example, conservative substitutions [defined as sets in Table 1 of Taylor, W.R., J. Mol. Biol. 188, 233 (1986)] in the pre-S1/S2/S amino acid sequence generally will not result in any substantial or novel modification of the principles and practice of the present invention. Conservative substitutions of hepatitis surface antigen are known; see Elfassi, E. et al., J. Theor. Biol. 121, 371 (1986). Alternatively, deletions within the S, pre-S1 or pre-S2 regions will not in general require any modifications of the processes for purification discussed herein. It will be understood that rHbsAg or surface antigen or portion thereof in this application includes any such variations in the amino acid sequence, whether by conservative amino acid

substitution, deletion, or other process, provided that rHbsAg, or the surface antigen, or portion thereof, is immunochemically reactive with antibodies specific for the S protein, the 22 nm particle, Australia antigen, Dane particles or other natural form of the hepatitis surface antigen sequence.

Many yeast expression systems are clearly adequate for providing sources of the rHbsAg. The S. cerevisiae expression system in Example 1 is intended as a merely incidental source. Other yeast vectors include but are not limited to shuttle vectors, cosmid plasmids, chimeric plasmids, and those having sequences derived from 2-micron circle plasmids. A variety of promoters and other yeast transcriptional control sequences may be used to express the inserted DNA sequence coding for surface antigen, including those of GAL10 and the alpha mating factor.

The genus Saccharomyces is composed of a variety of species. Most commonly used is Saccharomyces cerevisiae, or baker's yeast, as a host for the recombinant DNA-mediated expression of a variety of foreign polypeptides. However, the distinctions among other species of the genus Saccharomyces are not always well-defined. Many of these species are capable of interbreeding with S. cerevisiae and are likely to possess regulatable promoters which are analogous or identical to promoters is S. cerevisiae, including but not limited to GAL10, ADH2, GAP and/or alpha mating factor promoters. Therefore, it will be apparent to those skilled in the art that, for the expression of pre-S-containing polypeptides, the selection of a host strain extends to other species of the genus Saccharomyces, including but not limited to carlsbergensis, uvarum, rouxii, montanus, kluyveri, elongisporus, norbensis, oviformis, and diastaticus.

Several yeast genera, such as Hansenula, Candida, Torulopsis, and Pichia, have been shown to contain similar metabolic pathways for the utilization of methanol as a sole carbon source for growth. The gene for alcohol oxidase, an enzyme which participates in this metabolic pathway, has been isolated from Pichia pastoris. The P. pastoris alcohol oxidase promoter has been isolated and shown to be susceptible to methanol induction of expression. Such an inducible promoter is useful for the expression of polypeptides which are negatively selected in yeast. In particular, this promoter has been shown to be active on a plasmid for the inducible expression of the S domain in P. pastoris in particulate form. This observation highlights the ability of other yeast genera to function as hosts for the recombinant DNA-mediated expression of S polypeptides in immunologically active form. Therefore, it will be apparent to those skilled in the art that, for the expression of rHbsAg, the selection of a host strain extends to species from other genera of yeast from the Families Saccharomycetaceae and Cryptococcaceae, including, but not limited to Pichia, Candida, Hansenula, Torulopsis, Kluyveromyces, and Saccharomycopsis.

In the process of purifying hepatitis surface antigen, excessive instability and breakdown of the surface antigen has been observed. To combat this, solvents are typically buffered and also contain protease inhibitors such as PMSF and EDTA. To further reduce proteolysis, it is preferred to carry out all purification steps at about 4° C.

Typical protease inhibitors suitable for the procedures and protocols of this invention include but are not limited to metal-chelating agents, heavy metal ions, SH blocking reagents, substrate-like compounds of proteases or polypeptides that inhibit proteases. Some useful protease inhibitors are provided as follows:

$Ag^{++}$, $Hg^{++}$, $Cu^{++}$ and other heavy metal ions
antithrombin III
antithrombin III - heparin
$\alpha_1$-antitrypsin
aprotinin
basic amino acids
benzamidine
bestatin
$\alpha,\alpha'$-bipyridyl, Na-fluoride
4-bromo-phenacyl bromide
chicken egg white trypsin inhibitor
chymostatin
citrate
cysteine
4-dinitrophenol diethylphosphate
DFP (diisopropylphosphofluoridate)
DTT
E-64 (Boehringer Mannheim)
EDTA and other chelating agents
formaldehyde
guanidinium chloride

heparin
hirudin
4-hydroxymercuribenzoate
iodoacetamide
iodoacetic acid
leupeptin
$\alpha_2$-macroglobulin
mercaptoethanol
p-mercuribenzoate
mercuric chloride
$\alpha$-microglobulin
$\alpha$-N-(p-nitrobenzyl-oxycarbonyl)-L-arginylchloromethyl ketone
oxalate
pepstatin from a variety of sources, including Streptomyces
1,10-phenanthroline
2-phenanthroline
phenothiazine-N-carbonyl chloride
phosphoramidone
PMSF
pryrophosphate
SH-blocking reagents
silver nitrate
soybean trypsin inhibitor
p-toluene sulfonyl fluoride
TLCK[L-1-chloro-3-(4-tosylamido)-7-amino-2-heptanone-hydrochloride]
TRITON X-100 and other detergents at low concentrations
TPCK[L-1-chloro-3-(4-tosylamido)-4-phenyl-2-butanone]-
trypsin inhibitor from hen egg
ZPCK (benzyloxycarbonyl-L-phenylalanine)

Buffered solutions containing one or more of the listed inhibitors may be adapted to one or more steps in the process of purifying surface antigen.

## PURIFICATION

Yeast cells transformed with expression vectors coding for pre-S1/S2/S protein or variants thereof, are grown and harvested. If storage of the cells is desired, the cells are then washed in buffer, e.g. PBS, to form a cell paste. Storage of the cell paste is typically done by freezing in liquid $N_2$.

Purification typically begins as follows. A batch of frozen cell paste is thawed and suspended in buffer containing proteolytic inhibitors, e.g. PBS in 2mM PMSF, or hypertonic phosphate buffers. Cells are then disrupted, preferably by mechanical means. The gentle bead breakage method for disruption has been found unsuitable for scale-up. Disruption by a cell disrupter is the most preferred because of its rapid operation.

Yeast cell disruption results in a crude extract. It is necessary at this point to remove cell debris in the extract so that mechanical clogging in subsequent purification steps is avoided. Centrifugation at about 4,400 x g for 5 minutes has been found adequate, but it is readily understood that different centrifugation speeds for different times are also possible and easily rendered operable. The presence of pre-S polypeptide epitopes can be ascertained by the binding of these epitopes to polymerized human serum albumin (pHSA) in a sandwich RIA assay with labelled antibody specific to HBV surface antigen.

Further steps in the purification process are performed on the 4,400 x g supernatant remaining after the cell debris is removed from the crude extract by centrifugation at 4,400 x g for 5 minutes, as described above.

## SOLUBILIZATION

After the cell debris has been removed from the crude extract, the solubilization reaction can be conducted. Solubilization may also be undertaken at any other step subsequent to debris removal. For example, solubilization in urea-DTT may be performed after the additional step of adsorption and the elution from silica gel, or after another additional step of hydrophobic chromatography. These and other variations in the purification process are encompassed by the present invention, as long as solubilization of rHbsAg occurs at some point. It is preferred, however, to solubilize immediately after debris removal to enhance manipulations by early removal of insoluble precipitates in the purification process.

Solubilizing is conducted in the presence of one or more denaturing agents and one or more reducing agents, or mixtures thereof. The preferred selection of reagents is urea and dithiothreitol (DTT), in the concentration ranges of between about 1M-8M and 1-10mM, respectively. The most preferred solution for solubilization contains about 4M urea and about 5mM DTT. Buffers or protease inhibitors may be added as desired.

Generally, any denaturant of formula I is suitable and feasible for the purposes of solubilization:

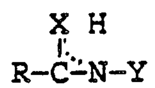

I

wherein
R is amino, loweralkylthio, loweralkyloxy, or sulfur;
X is amino, sulfur or oxygen; and
Y is hydrogen or amino.
This formula includes urea, wherein R is amino, X is O and Y is H. Other denaturants of use in alternative solubilization protocols include, but are not limited to, detergents of the nonoxynol series, octoxynol series, polyoxyethylene alcohol series, polyoxyethylene (20) sorbitan mono-oleate series, deoxycholate, or octylglucopyranoside, and the like. Zwitterionic detergents are also useful and suitable agents.

Reducing agents useful for solubilizing rHbsAg are thiol reagents that will not modify substantially the SH groups of the protein rHbsAg. Cysteine, homocysteine, $\beta$-mercaptoethanol, dithiothreitol, dithioerythritol and sodium bisulfite are some of the reagents that fall within the category of such thiol reagents. On the other hand, Ellman's reagent and sodium borohydride are each regarded as too strong for the solubilization reaction, since reaction of rHbsAg with each yields a product of substantially irreversibly modified character not subject to reversible rearrangement and formation of new disulfide linkages among rHbsAg cysteine residues.

## ADDITIONAL STEPS

Other conventional or known steps normally used in purification of recombinant proteins may be added to the process of purifying rHbsAg. These steps include, but are not limited to:

(a) selective adsorption or partition on a solid-phase, e.g. silica gel, calcium phosphate charcoal, or celite alumina;

(b) hydrophobic chromatography with, e.g. butyl agarose, hexyl agarose, octyl agarose or phenyl agarose; and

(c) selective extraction with solvents or reagents that release undesired membrane-bound proteins followed by other solvents or reagents that release membrane bound rHbsAg. Extraction with other solvents or reagents for other purposes may be needed in different steps.

(d) Precipitation is another step useful for isolating rHbsAg. It can be performed with salts such as ammonium sulfate, or on a pH gradient by isoelectric precipitation. Other methods include

(e) chromatography by any standard method, including paper, thin-layer, gel, molecular sieve, molecular exclusion, ion-exchange, ligand affinity, immunoaffinity, or by electrophoresis;

(f) solvent fractionation by two phase extractions, e.g. with PEG and dextran [Anderson, E. et al., Ann. N.Y. Acad. Sci. 413, 115 (1983)].

(g) dialysis, ultrafiltration, or diafiltration;

(h) density-gradient centrifugation;

(i) electrofocusing;

(j) freeze drying, lyophilization; or

(k) crystallization.

This list is by no means exhaustive. Its order is not an indication of the preferred order of purification. It will be understood that a successful purification of rHbsAg may be conducted with any or all of steps (a)-(k), and that in principle solubilization as described above may be inserted as an additional step at any point.

In the examples the following materials were purchased from commercial sources: Urea and guanidine• HCl, Schwartz/Mann Biotech; TRITON X-100, Fisher Scientific Company; dithiothreitol, Bio-Rad.

Throughout this application, glycoprotein was determined by the periodic acid-Schiff base procedure of Neville. D. M. et al., Methods in Enzymology 32, 92 (1974). Protein concentrations were determined by the SDS-Lowry assay, e.g. Lowry, 0. H. et al., J. Biol. Chem. 193, 265 (1951); and the Coomassie blue-binding assay, e.g. Bradford, M.M., Anal. Biochem. 72, 248 (1976).

Immunochemical tests were performed on the lots purified according to the following procedures. Binding to polymerized human serum albumin (pHSA) was conducted according to Valenzuela et. al., Biotechnology, 3, 317 (1985). Immunochemical determinants were assayed by a variety of conventional procedures, including AUSRIA® II-125 (Abbott Labs), and AUSAB® (Abbott Labs). Biological activity was tested by the mouse potency test.

## EXAMPLE 1

### Preparation of partially purified rHbsAg

Samples of partially purified recombinant surface antigen were prepared substantially by the procedures of Wampler, D.E. et al., Proc. Natl Acad. Sci. 82, 6830 (1985) through the step involving hydrophobic chromatography on butyl-agarose, yielding butyl-agarose product. These procedures are as follows:

Two yeast strains were used as a source of rHbsAg. The first is that described by Valenzuela et al. Nature 298, 347 (1982). The second used a glyceraldehyde-3-phosphate dehydrogenase (GAP) promoter instead of the alcohol dehydrogenase (ADH) promoter. Cells were grown as described by Carty et al., Eighty-Fourth ASM Meeting, March 12, 1984, St. Louis, MO, abstr 030, p. 194, and harvested. The cell paste was suspended in an equal volume of hypertonic phosphate buffer (0.1 M sodium phosphate, pH 7.2/0.5 M NaCl). Phenylmethylsulfonyl fluoride (0.2 M in isopropanol) was added to a final concentration of 2mM and the cells were disrupted by seven or nine passes through a laboratory homogenizer for 15 minutes at 10,000 psig yielding a crude extract. The crude extract (32-70 mg of protein per ml) was diluted with 4 vol of 0.01 M phosphate buffer (pH 7.5) containing 0.1% TRITON X-100 (Rohm and Haas). Cell debris was removed by continuous-flow centrifugation, and the supernatant solution was concentrated 5-fold in a hollow fiber unit and diafiltered with 5 vol of phosphate-buffered saline (7mM sodium phosphate, pH 7.2/0.15 M NaCl). TRITON X-100 was removed by using an XAD-4 beads cartridge [Rohm and Haas, see Cheetham, P.S.J. Anal. Biochem. 92, 447 (1979)]. HBsAg was partially purified by adsorption and elution with 50 mM sodium borate buffer, pH 8.7, from fused silica [Pillot, J. et al., J. Clin. Microbiol. 4, 205 (1976). The eluate was clarified by centrifugation for 35 minutes at 11,000 x g at 4° C, then routinely stored at 4° C. For removal of insoluble precipitates at this stage, centrifugation at 8000 x g for 4 minutes was performed and the resulting clarified product was further purified by adsorption on butyl-agarose, yielding the butyl-agarose product as eluate.

## EXAMPLE 2

### Solubilization

The butyl-agarose product was treated with urea-DTT or KSCN, or both, as follows:

(a) Urea-DDT treatment. A sample of the butyl-agarose product of Example 1 was incubated for 15-20 minutes at 4° C in 4M Urea and 5 mM DTT. The impurities dissociated from the antigen were separated from the antigen by diafiltration over a hollow fiber membrane (100,000 NMW, 0.6 $ft^2$, 5 psi) with 5 vol. of 2M Urea in 2 mM DTT, followed by 10 volumes of PBS, yielding retentates treated with Urea-DTT.

(b) KSCN treatment. Butyl-agarose product samples of Example 1, or urea-DTT treated samples of step (a) of this Example, were incubated in 3M KSCN in PBS at 4° C for 16 hours. The impurities dissociated from the antigen were then removed by diafiltration over a hollow fiber membrane (100,000 NMW, 0.6 $ft.^2$) with 5 volumes of 3M KSCN in PBS followed by 10 volumes of PBS, yielding samples treated with KSCN.

Samples treated with Urea-DTT or KSCN, or both, were examined under electron microscopy and found to be 20 nm particles of typical appearance. These samples were then analyzed for immunological potency by the mouse potency test.

Mouse potency was assessed by the procedures as follows. Antigen samples were prepared for testing by subjecting to sterile filtration with a 0.22 µm membrane, then treating a sample (20 µg/ml protein) with 1:4,000 formalin for 72 hours at 37° C. The formalin-treated sample was then adsorbed on alum by adding 0.085 ml of a sterile 5.44% solution of potassium aluminum sulfate, with stirring, to each ml of sample and adjusting the pH of the mixture to 6.75 with 1 N NaOH. After stirring for two hours, the suspension was centrifuged 10 minutes at 1000 x g. The supernatant liquid was decanted from the alum precipitate and discarded. The alum adsorbed rHbsAg was resuspended in physiological saline to the original volume and thimerosal was added to a concentration of 1:20,000. The preparation was tested in mice undiluted and diluted in alum placebo to contain 0.025, 0.006 and 0.0016 µg/ml. One milliliter of each preparation, or alum placebo, was injected intraperitoneally into each of 10 mice. The mice were individually bled 28 days later and antibody titers were measured by the AUSAB ® radioimmune assay (Abbott). Data were analyzed to determine seroconversion rates to the different doses of subunit HBsAg. A probit analysis was performed, plots of empirical probit versus dose were made, iterative maximum likelihood least squares performed on the data and the $ED_{50}$ values (µg) were obtained.

Results of the mouse potency tests are summarized in the table as follows. Treatment with urea-DTT substantially improved mouse potency.

**TABLE**

**Mouse Potency Tests of rHbsAg**

| Lot No. | Starting Material | Treatment: Urea-DTT | Treatment: KSCN[a] | Mouse Potency[b] $ED_{50}$ (µg) |
|---|---|---|---|---|
| 87706 | Butyl agarose product | No | No | 1.01 |
| | | No | Yes[c] | 0.74 |
| | | Yes[d] | No | 0.44 |
| | | Yes[d] | No | 0.82 |
| | | Yes | Yes | 0.57 |
| 87708 | Butyl agarose product | Yes | Yes | 0.36 |
| 87708 | Pre-Sepharose 6B pooled (waste) fraction[e] | No | Yes | 1.36 |
| | | Yes | No | 1.11 |
| | | Yes | Yes | 0.98 |

TABLE (con't)

Mouse Potency Tests of rHbsAg

| Lot No. | Starting Material | Treatment Urea-DTT | KSCN[a] | Mouse Potency[b] $ED_{50}$ (mg) |
|---|---|---|---|---|
| 87712 | Butyl agarose product | | | |
| | | No | Yes | 0.48 |
| | | Yes | No | 0.52 |
| | | Yes | Yes | 0.41 |
| 87714 | Butyl agarose product | | | |
| | | No | Yes | 0.58 |
| | | Yes | No | 0.52 |
| | | Yes | Yes | 0.47 |

a When samples were treated with urea-DTT and KSCN, urea-DTT treatment was always conducted first, followed by KSCN treatment.

b $ED_{50}$ is the amount of antigen in mg required to seroconvert 50% of the mice.

c KSCN was removed by dialysis against PBS (12,000 MWCO) instead of the diafiltration method described in Example 2 (b).

d These samples were treated with Urea-DTT then chromatographed on a BIO-GEL A-5 m column to remove impurities, before conducting the mouse potency test, as follows: A sample of butyl-agarose product was treated with urea-DTT as described in Example 2(a). The antigen preparation was then applied on a BIO-GEL A-5m (2.5 x 3.6 cm) column equilibrated with 2mM DTT in 2M Urea, and eluted with 2mM DTT in 2M

**Urea from the column as a single AUSRIA* positive peak. This peak fraction was pooled and then dialyzed against PBS in a bag (12,000 MWCO), yielding lot 87706 treated with urea-DTT.**

e **This fraction is the collected peak of high $OD_{280}$ absorption collected after a subsequent purification of the butyl agarose product on SEPHAROSE 6B.**

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims.

## Claims

1. A method for solubilizing recombinant hepatitis B surface antigen from a precipitated, partially purified recombinant hepatitis antigen obtained from a recombinant expression system, comprising the steps of:

(a) treating a preparation of precipitated, partially purified recombinant hepatitis B antigen, or portion of said antigen thereof, with a quantity of denaturing agent in the presence of a reducing agent effective to solubilize said preparation; and

(b) removing the denaturing agent and the reducing agent, yielding a preparation of substantially soluble, partially purified recombinant hepatitis antigen, or portion of said antigen thereof.

The method of claim 1 wherein the partially purified recombinant hepatitis antigen, or portion of said antigen thereof, has been subjected, prior to step (a) of claim 1, to the steps of

(a) growing yeast cells expressing rHbsAg, or portion thereof;

(b) harvesting said cells;

(c) disrupting said cells and removing the debris, yielding partially purified rHbsAg.

3. The method of claim 1 wherein the partially purified recombinant hepatitis antigen, or portion of said antigen thereof, has been subjected, prior to step (a) of claim 1, to the steps of

(a) growing yeast cells expressing rHbsAg, or portion thereof;

(b) harvesting said cells;

(c) disrupting said cells and removing the debris, yielding a partially purified, crude extract; and

(d) subjecting the crude extract to adsorption and elution from fused silica, yielding a partially purified rHbsAg.

4. The method of claim 1 wherein the partially purified recombinant hepatitis antigen, or portion of said antigen thereof, has been subjected, prior to step (a) of claim 1, to the steps of

(a) growing yeast cells expressing rHbsAg, or portion thereof;

(b) harvesting said cells;

(c) disrupting said cells and removing the debris, yielding a partially purified, crude extract;

(d) subjecting the crude extract to adsorption and elution from tested silica, yielding a partially purified eluate; and

(e) subjecting the eluate to hydrophobic chromatography, yielding a partially purified rHbsAg.

5. The method of claims 1 or 2 or 3 or 4, wherein the denaturing agent is urea.

6. The method of claims 1 or 2 or 3 or 4, wherein the reducing agent is dithiothreitol.

7. The method of claims 1 or 2 or 3 or 4, wherein the denaturing agent is urea and the reducing agent is dithiothreitol.

8. The method of claim 7, wherein the urea is at a final concentration of about 4M and the dithiothereitol is at a final concentration of about 5mM.